# NEUE EUROPÄISCHE PATENTSCHRIFT

(11) **EP 0 731 685 B2**
(45) Veröffentlichungstag und Bekanntmachung des Hinweises auf die Entscheidung über den Einspruch: **05.12.2007**
(45) Hinweis auf die Patenterteilung: 12.07.2000
(21) Anmeldenummer: 95903303.6
(22) Anmeldetag: 29.11.1994
(51) Int. Cl.: A61K 9/113

(54) **STABILE MULTIPLE X/O/Y-EMULSION**
STABLE MULTIPLE X/O/Y EMULSION
EMULSION X/O/Y MULTIPLE STABLE

(30) Priorität: 02.12.1993 DE 4341113
(43) Veröffentlichungstag der Anmeldung: 18.09.1996
(73) Patentinhaber: IFAC Institut für angewandte Colloidtechnologie GmbH & Co. KG, 47138 Duisburg (DE)
(72) Erfinder: DAHMS, Gerd, H., D-47166 Duisburg (DE)
(74) Vertreter: Bublak, Wolfgang
(86) Internationale Anmeldenummer: PCT/EP1994/003955
(87) Internationale Veröffentlichungsnummer: WO 1995/015143

(56) Entgegenhaltungen:
- EP-A- 0 345 075
- EP-A- 0 614 660
- EP-B- 10 345 075
- EP-B- 10 407 834
- EP-B- 20 065 929
- WO-A-94/01073
- GB-A- 1 541 463
- GB-A- 2 242 358
- US-A- 4 626 443
- US-A- 4 626 444
- US-A- 4 714 566
- US-A- 4 931 210
- US-A- 5 304 370
- S. MAGDASSI: 'Correlation between nature of emulsifier and multiple emulsion stability' DRUG DEVELOPMENT AND INDUSTRIAL PHARMACY Bd. 11, 1985, Seiten 791 - 798, XP002972972
- PATENT ABSTRACTS OF JAPAN vol. 8, no. 246 (C-251) (1683) 10. November 1984 & JP,A,59 127 646 (KOBAYASHI KOOSEE K.K.) 23. Juli 1984
- M. FRENKEL: 'Multiple Emulsions' JOURNAL OF COLLOID AND INTERFACE SCIENCE Bd. 94, Nr. 1, Juli 1983, Seiten 174 - 178, XP002972973
- Kamlesh P.Oza and Sylvan G.Frank:"Drug Release from Emulsions Stabilized by Colloidal Microcrystalline Cellulose".J.Dispersion Science and Technology, 10(2), 187-210(1989)
- S.Matsumoto et al:"Preparation of W/O/W Emulsions in an Edible Form on the basis of Phase Inversion Technique ".J.Dispersion Science and Technology.6(5),507-521 (1985)
- McCutcheon's, volume 1:Emulsifiers & Detergents,International Edition 2000,pp.2,23,38,40,134,174
- International Cosmetic Ingredient Dictionary and Handbook,7th edition, volume 1(Mica-Microcrystalline)
- S.Friberg: "Liquid Crystals and Emulsions".Advances in Liquid Crystals, vol.2,S3 173-197
- K.-H.Schrader:"Grundlagen und Rezepturen der Kosmetika",2., verbesserte und erweiterte Auflage, Hüthig Buch Verlag Heidelberg,1989, S 58-60, 103-104,113-115,433,435,440,451,456

## Beschreibung

Die vorliegende Anmeldung betrifft eine stabile multiple Emulsion vom X/O/Y-Typ.

Stabile multiple Emulsionen vom W/O/W-Typ sind beispielsweise aus der DE-OS 41 31 678 bekannt und werden in mehreren Literaturveröffentlichungen beschrieben, z.B. A.T.Florence et al.,"The Formulation and Stability of Multiple Emulsions", Int.J.Pharmaceutics,Bd.11,277-308,1982.

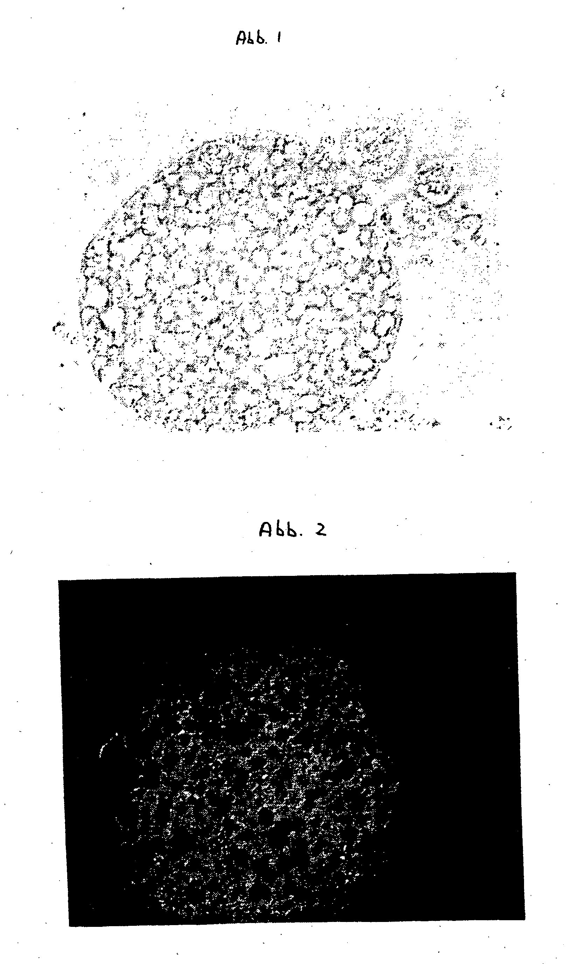

In der Veröffentlichung "Correlation between Nature of Emulsifier and Multiple Emulsion Stability" von S.Madgassi et al.,Drug Development and Industrial Pharmacy, Bd. 11,791-798,1985, sind multiple W/O/W-Emulsionen beschrieben, deren interne wäßrige Phase einen Emulgator mit einem HLB-Wert von 6 und deren äußere wäßrige Phase einen ölähnlichen Emulgator mit einem HLB-Wert von 10-12 enthält. Die Stabilität der so erhaltenen W/O/W-Emulsionen ist relativ gering und übersteigt nach Fig.1 und 2 kaum einen Monat.

In der weiteren Veröffentlichung "Multiple Emulsions" von M. Frenkel et al.,J.Colloid Interface Sci.,Bd.94,174-178,1982, sind multiple Emulsionen aus einer internen waßrigen Phase, die einen Emulgator mit einem HLB-Wert von 2-6 enthält, und einer externen wäßrigen Phase beschrieben, die einen Emulgator mit einem HLB-Wert von 10-14 enthält. Die Verhältnisse sind so gewählt, daß ein berechneter "gewichteter" HLB-Wert für beide Emulgatoren im Bereich von 7,7-10,5 liegt. Die so hergestellten multiplen Emulsionen werden ohne nähere Angaben als "stabil" beschrieben, sofern die Tröpfchengröße über 5 µm liegt.

In der EP-A-0 345 075 sind Emulsionen vom W/O/W-Typ beschrieben, die in der internen wäßrigen Phase einen Emulgator mit einem HLB-Wert nicht größer als 6, vorzugsweise nicht größer als 5, und in der äußeren wäßrigen Phase einen Emulgator mit einem HLB-Wert von wenigstens 8 enthalten. Zusätzlich ist in der internen wäßrigen Phase eine Komponente zur Einstellung des osmotischen Drucks und in der externen wäßrigen Phase ein Gelbildner enthalten.

Allgemein besteht danach eine multiple W/O/W-Emulsion aus einer W/O-Komponente, d.h. einer Wasserin-Öl-Emulsion aus in einer hydrophoben Flüssigkeit dispergierten Tropfen einer hydrophilen Flüssigkeit, und einer wäßrigen Komponente, in der diese W/O-Komponente verteilt ist. Die interne Phase der W/O-Komponente kann beispielsweise aus einer wäßrigen Lösung gebildet sein. Die hydrophobe externe Phase der W/O-Komponente ist gewöhnlich aus Siliconöl, Paraffinöl, Triglycerid, Fettalkohol, Esteröl oder dergleichen, sowie deren Mischung ausgewählt.

Eine solche multiple Emulsion kann nur unter Zuhilfenahme von Emulgatoren hergestellt werden. Nach einem allgemein anerkannten Auswahlsystem, vgl. Encyclopedia of Emulsion Technology, Ed. P.Becker, Marcel Dekker, New York 1988, werden die Emulgatoren gemäß einem HLB-Wert nach dem Verhältnis ihrer hydrophilen und hydrophoben Anteile unterschieden; der HLB-Wert ergibt sich aus den durch 5 geteilten, prozentualen hydrophilen Anteilen am Gesamtmolgewicht des Emulgators. Man unterscheidet dabei Emulgatoren zur Bildung von W/O-Emulsionen mit einem HLB-Wert im Bereich von 3 bis 6, Emulgatoren als Netzmittel mit einem HLB-Wert im Bereich von 7 bis 9 und Emulgatoren zur Bildung von O/W-Emulsionen mit einem HLB-Wert im Bereich von 8 bis 18.

Bei W/O/W-Emulsionen der eingangs genannten Art soll die wäßrige Phase als Träger für den Transport der Tropfen der W/O-Phase an den Ort ihrer Wirkung dienen, ohne daß der in den Tropfen enthaltene Stoff sich mit dem Wasser mischen oder damit in Berührung kommen kann. Wichtige Kriterien dafür sind
1. Stabilität der W/O-Phase gegen Temperatureinflüsse;
2. Stabilität der W/O-Phase gegen Koaleszenz;
3. Unempfindlichkeit gegen die bei der Herstellung der Emulsion auftretenden Scherkräfte; und
4. Unabhängigkeit der Stabilität vom Volumenverhältnis zwischen der W/O-Phase und der wäßrigen Phase.

Die bisher bekannten multiplen Emulsionen genügen diesen Anforderungen nur unvollkommen; Verbesserungen konnten bisher dadurch erzielt worden, daß verhältnismäßig komplizierte Verfahrensschritte bei der Herstellung der Emulsion durchlaufen werden mußten, um die gewünschten Stabilitätsanforderungen zu erfüllen, oder daß besonders ausgesuchte und zusammengesetzte Emulgatoren eingesetzt werden mußten, um die gewünschte Stabilität zu erzielen. Nach der eingangs erwähnten DE-OS 41 31 678 ist ein Gemisch von Emulgatoren erforderlich, deren einer ein W/O-Emulgator mit einem HLB-Wert im Bereich von weit unter 3 bis nahe 5 und deren anderer ein O/W-Emulgator mit einem HLB-Wert im Bereich von 10 und weit darüber ist.

Bei den bisher bekannten W/O/W-Emulsionen sind immer nur rein wäßrige Phasen und Ölphasen verwendet worden. Der Einsatz von nichtwäßrigen Phasen ist dabei nicht in Betracht gezogen worden.

Dementsprechend besteht die Aufgabe der Erfindung darin, eine einfach zusammengesetzte multiple Emulsion anzugeben, die auf einfache Weise hergestellt werden kann und hinsichtlich der vorgenannten Stabilitätskriterien und in ihrer Anwendbarkeit gegenüber den bekannten Emulsionen vom W/O/W-Typ signifikant verbessert ist.

Insbesondere soll die erfindungsgemäße stabile multiple Emulsion die Aufnahme von Feststoffen ermöglichen.

Zur Lösung dieser Aufgaben sieht die Erfindung eine stabile multiple Emulsion vom X/O/Y-Typ vor, bei der X eine mit Öl nicht mischbare Komponente ist und O eine Ölphase und Y eine wäßrige Phase sind und deren Emulgator oder Emulgatoren ausschließlich ausgewählt sind aus der Gruppe W/O-Emulgatoren, Emulgatoren mit einem HLB-Werts ≤6 und deren Mischung. Sie ist dadurch gekennzeichnet, daß die wäßrige Phase ein wässriges Gel bildet, wobei das Gel von einem ein flüssigkristallines Netzwerk bildenden Emulgator gebildet ist und daß die X-Komponente ein mit Wasser mischbares Polyol ist.

Vorteilhafterweise ist der Emulgator aus der Gruppe Glycerinester, Sorbitanester, Sorbitolester, Polyglycerinester, Fettalkohole, Propylenglycolester, Alkylglucosidester, Zuckerester, Lecithin, Siliconcopolymere und deren Mischungen oder Derivaten ausgewählt.

Die mit Hilfe mindestens eines dieser Emulgatoren bergestellten Emulsionen werden durch einen einfachen Mischvorgang unter Rühren erhalten, wobei die Stabilität dieser multiplen Emulsionen weder durch die eingetragene Rührwerksenergie, noch durch die Art des Rührwerkzeugs beeinflußt wird. Tatsächlich kann zur Herstellung der erfindungsgemäßen multiplen Emulsion jedes handelsübliche Rührwerk eingesetzt werden.

Die so hergestellten multiplen Emulsionen nach der Erfindung besitzen hohe Langzeitstabilität und erfüllen die üblichen Stabilitätsanforderungen im Temperaturbereich von -5°C bis +45°C. Auch im stark mit Wasser verdünntem Zustand (1:100) sind die multiplen Emulsionen nach der Erfindung stabil: Selbst nach Aggregation und Auf rahmen kommt es zu keiner Koaleszenz, denn das aufgerahmte Material kann durch einfaches Schütteln vollständig redispergiert werden, und die so wieder hergestellte Emulsion ist dann wieder genauso stabil wie die ursprünglich hergestellte Emulsion.

Die erfindungsgemäßen stabilen multiplen Emulsionen zeichnen sich insbesondere dadurch aus, daß die darin enthaltenen Tropfen eine Größenverteilung zwischen 1 µm und 10 µm und damit eine relativ geringe und kleinere Tropfengröße haben als die bekannten multiplen Emulsionen, was für viele Anwendungen von erheblichem Vorteil ist.

Insbesondere ist es aber möglich, nach der Erfindung mehrere X/O-Phasen getrennt nebeneinander in der wäßrigen Phase zu dispergieren, wobei ebenfalls Langzeitstabilität im vorgenannten Sinne gegeben ist und die Tropfen der verschiedenen X/O-Phasen vollständig voneinander getrennt bleiben und weder eine gegenseitige Durchmischung, noch ein Austausch oder dergleichen mit der äußeren wäßrigen Phase eintritt. Das ist von besonderer Bedeutung in allen Fällen, in denen die X/O-Phasen unterschiedliche Wirkstoffe enthalten, die z.B. in der Medizin oder in der Kosmetik getrennt, aber in einem gemeinsamen Träger zur Anwendung gebracht werden sollen.

Zur näheren Erläuterung der Erfindung werden nachstehend lediglich beispielhaft einige erfindungsgemäße stabile multiple Emulsionen beschrieben. Darin beziehen sich alle Prozentangaben auf Gewichtsprozente des jeweiligen Bestandteils, bezogen auf das Gesamtgewicht der multiplen Emulsion = 100. Die angegebenen Trivialnamen sind dem CTFA-Verzeichnis entnommen.

### BEISPIEL 1 Herstellung einer Primäremulsion (nicht erfinderisch)

Es werden unter Verwendung üblicher Gefäße und Rührwerke eine Ölphase und eine Wasserphase hergestellt und auf eine Mischtemperatur im Bereich von 20°C bis 90°C, vorzugsweise 60°C bis 80°C, aufgeheizt. Anschließend werden die aufgeheizte Ölphase und die aufgeheizte Wasserphase unter Rühren mit einem üblichen Rührwerk zusanmengegeben; nach der Zusammengabe wird eine weitere Minute gerührt. Anschließend wird die so erhaltene Emulsion auf Raumtemperatur abgekühlt oder abkühlen gelassen.

| Die Ölphase enthielt: | |
|---|---|
| Sorbitanmonostearat | 6,30% |
| Laurylmethicon-Copolyol | 2,70% |
| Paraffinöl | 10,00% |

| Die Wasserphase enthielt | |
|---|---|
| Demineralisiertes Wasser | 80,80% |
| Phenonip | 0,20% |

In den nachfolgenden Beispielen wird diese Emulsion als Primäremulsion bezeichnet; sie stellt eine multiple W/O/W-Emulsion dar und dient zur Herstellung von Emulsionen mit unterschiedlichen X/O-Phasen.

Die multiple Emulsion wurde wie vorstehend hergestellt; lediglich die Zusammensetzung war unterschiedlich.

| Die Öl-Phase enthielt | |
|---|---|
| Sorbitanmonostearat | 4,50% |
| Laurylmethicon-Copolyol | 4,50% |
| Paraffinöl | 10,00% |
| Cyclomethicon | 10,00% |

| Die Wasserphase enthielt | |
|---|---|
| Demineralisiertes Wasser | 70,80% |
| Phenonip | 0.20% |

### Herstellung einer multiplen Emulsion (nicht erfinderisch)

Dieses Experiment sollte dazu dienen, die Nichtmischbarkeit verschiedener X/O-Phasen durch mikroskopischen Beobachtung aufzuzeigen, wobei nachfolgend die entsprechenden Mikroskopaufnahmen wiedergegeben sind. Gleichzeitig belegt dieses Beispiel auch, daß in einer Phase der Emulsion auch fein verteilte Feststoffe wie Titandioxid verteilt sein können, die beispielsweise in kosmetischen Zubereitungen als Lichtschutzfilter dienen.

Eine Sekundäremulsion Nr. 1 bestand aus einer Ölphase und einer Wasserphase.

| Die Ölphase enthielt | |
|---|---|
| Laurylmethicon-Copolyol | 1,5% |
| Paraffinöl | 16,0% |
| TIOVEIL MOTG (Titandioxid in Mineralöl/Triglycerid dispergiert) | 10,0%; |

| die Wasserphase enthielt | |
|---|---|
| Wasser | 71,5% |
| Natriumchlorid | 1,0%: |

diese Sekundäremulsion bildet eine W/O-Emulsion.

Eine Sekundäremulsion Nr. 2 enthielt eine Ölphase und eine X-Komponente, nämlich

| | |
|---|---|
| Cyclomethicon + Dimethicon-Copolyol | 15% |
| Cyclomethicon | 30% |

| (Ölphase) und | |
|---|---|
| Propylenglycol (X-Komponente) | 55% |

und bildet somit eine X/O-Emulsion.

Die gewünschte X/O/W-Emulsion wird dann dadurch erhalten, daß die erste der vorstehend beschriebenen Primäremulsionen auf eine Temperatur im Bereich von 20°C bis 90°C, vorzugsweise 50°C bis 60°C, erwärmt wird; anschließend werden unter Rühren die Sekundäremulsionen Nr. 1 und Nr. 2 nacheinander zugegeben, und die Mischung wird auf Raumtemperatur abgekühlt.

In entsprechender Weise werden diese Primäremulsion und die Sekundäremulsion Nr. 1 zusammengegeben. Nach Abkühlen auf Raumtemperatur werden die Proben mikroskopiert; auf den in den nachfolgenden Abbildungen wiedergegebenen Mikroskopaufnahmen sind die nebeneinander getrennt vorliegenden Tropfen der Primäremulsion und der Sekundäremulsion deutlich zu erkennen. Diese Struktur bleibt auch nach längerer Lagerzeit erhalten; auch nach Aggregation und Aufrahmen und anschließendem Redispergieren ist keine Änderung erkennbar.

Die nachfolgende Abbildung 1 zeigt die X/O/Y-Emulsion in Phasenkontrast bei 600-facher Vergrößerung. Man erkennt darin einen groBen Tropfen der Ölphase der Sekundäremulsion Nr. 1, der von einer Wasserphase umgeben ist, die aus der Wasserphase der Primäremulsion besteht. Innerhalb des großen Tropfens erkennt man die unstrukturierten Tröpfchen der Wasserphase der Sekundäremulsion Nr. 1. Diese unstrukturierten Tröpfchen befinden sich in der titandioxidhaltigen Ölphase der Sekundäremulsion Nr. 1. Außerhalb des großen Öltropfens befinden sich kleine Öltropfen, welche von der Ölphase der W/O/W-Primäremulsion gebildet sind; darin sind kleine Wassertropfen in Form dunkler Punkte erkennbar verteilt. Die beiden Arten von Öltropfen liegen deutlich getrennt nebeneinander vor, und man sieht, daß die beiden Ölphasen sich nicht miteinander mischen.

Abbildung 2 zeigt die gleiche Mikroskopaufnahme in polarisiertem Licht. Unter diesen Bedingungen erscheinen die unstrukturierten Tropfen der Wasserphase der Primäremulsion dunkel, während die Titandioxidteilchen im Öltropfen der Sekundäremulsion Nr. 1 deutlich aufscheinen. Die außerhalb des großen Öltrpfens befindlichen kleinen Öltropfen erscheinen wenig strukturiert und sind frei von den Titandioxidteilchen; sie geben sich dadurch als Öltropfen der Primäremulsion zu erkennen, in denen Wassertropfen undeutlich sichtbar sind.

Gleiche Ergebnisse werden erhalten, wenn der Emulgator Laurylmethicon-Copolyol in der Sekundäremulsion Nr.1 und/oder der Emulgator Dimethicon-Copolyol durch in der Sekundäremulsion Nr. 2 durch irgendeinen anderen Emulgator aus der Gruppe der vorstehend auf Seite 6 genannten Emulgatoren ersetzt wird.

### BEISPIEL 1 (erfinderisch)

Dieses Beispiel soll zeigen, daß die multiplen Emulsionen als Gel vorliegen.

Dazu wird zunächst ein flüssig-kristallines Gel aus einer gelbildenden Emulgatorphase und einer Wasserphase hergestellt. Die Emulgatorphase und die Wasserphase werden getrennt auf 80°C erwärmt. Anschließend wird die Emulgatorphase unter Rühren in die Wasserphase gegeben und die Mischung unter weiterem Rühren auf Raumtemperatur abgekühlt.

| Die Gelphase enthielt | |
|---|---|
| Methylglucosiddistearat | 10,0% |
| Wasser | 89,8% |
| Phenonip | 0,2% |

Das so erhaltene flüssig-kristalline Gel wird auf eine Temperatur im Bereich von 20°C bis 90°C, vorzugsweise im Bereich von 50°C bis 60°C, erwärmt und nacheinander mit den Sekundäremulsionen Nr. 1 und Nr. 2 unter Rühren vermischt. Die Bestandteile wurden in den folgenden Mengen in Gewichtsprozent verwendet, jeweils bezogen auf das Gewicht des Gesamtproduktes = 100:

| | |
|---|---|
| Flüssig-kristallines Gel | 70% |
| Sekundäremulsion Nr. 1 | 20% |
| Sekundäremulsion Nr. 2 | 10% |

### BEISPIEL 2

Die nach Beispiel 1 erhaltene flüssig-kristalline Phase wird auf eine Temperatur im Bereich von 20°C bis 90°C, vorzugsweise 40°C bis 60°C, erwärmt; anschließend wird die erwärmte flüssig-kristalline Phase nacheinander unter Rühren mit den Sekundäremulsionen Nr. 1 und Nr. 2 und der ersten der Primäremulsionen versetzt, und zwar in den nachstehend angegebenen Mengen in Gewichtsprozent, jeweils bezogen auf das Gewicht des Gesamtproduktes = 100:

| | |
|---|---|
| Flüssig-kristalline Phase | 80% |
| Sekundäremulsion Nr. 1 | 10% |
| Sekundäremulsion Nr. 2 | 5% |
| Primäremulsion | 5% |

Die vorgenannten stabilen multiplen Emulsionen sind wegen ihrer Stabilität und wegen der Beständigkeit der darin vorhandenen Tropfen in besonderem Maße als Träger für viele Arten von Wirkstoffen geeignet, wobei gemäß den Beispielen der Träger ein flüssig-kristallines Gel ist.
Als einfacher Anwendungsfall sei im folgenden erwähnt, daß es wünschenswert ist, wenn Waschmittel für eine Waschmaschine in flüssiger Form vorliegen, weil flüssige Waschmittel leichter als feste Waschmittel dosiert werden können. Weiterhin ist es wünschenswert, dem Waschmittel Enzyme wie Proteasen, Lipasen und Amylasen zuzusetzen. Üblicherweise sind diese Enzyme aber in wäßriger Lösung nicht langzeitbeständig.

Die Herstellung von Emulsionen mit verschiedenen X/O-Phasen ermöglicht auch, miteinander unverträgliche Wirkstoffe gemeinsam in einem einzigen Träger unterzubringen. Beispielsweise in der Kosmetik ist es oft erwünscht, miteinander unverträgliche Wirkstoffe wie Collagenpräparate und Harnstoff zur Anwendung auf die Haut aufzubringen. Das ist unter normalen Umständen bei Verwendung eines wäßrigen Trägergels nicht möglich. Unter diesen Umständen würde nämlich das Collagenpräparat in wäßriger Phase durch Harnstoff denaturiert und dadurch seine Wirkung verlieren, vgl. H. Lindner "Kollagen in der Kosmetik", Parfümerie und Kosmetik Bd. 65, 340-343; 1984. Werden die beiden Wirkstoffe jedoch getrennt voneinander auf die Haut auf getragen, so kommt es nicht zu dieser Denaturierung. Dies kann durch die Verwendung einer Emulsion entsprechend Beispiel 4 erreicht werden:

Beide Wirkstoffe sind unter diesen Bedingungen in einem gemeinsamen Trägergel verteilt, wobei wiederum der Emulgator verhindert, daß die Inhaltsstoffe der beiden X/O-Phasen in Kontakt oder Wechselwirkung miteinander oder mit dem Trägergel kommen. So wird entsprechend Beispiel 1 ein Trägergel auf flüssig-kristalliner Basis hergestellt; wie vorstehend wird eine Sekundäremulsion Nr. 1 erhalten, indem in der Wasserphase Harnstoff anstelle von Natriumchlorid gelöst wird und in der Ölphase die Titandioxiddispersion entfällt; entsprechend der Sekundäremulsion Nr. 2 wird das Collagenpräparat in der Polyolphase gelöst und diese in der betreffenden Ölphase dispergiert. Das nach Beispiel 1 hergestellte Trägergel enthält dann die beiden Wirkstoffe in den voneinander getrennten, nicht im gegenseitigen Austausch oder im Austausch mit dem wäßrigen Trägergel stehenden Öltropfen der verschiedenen X/O-Phasen, sodaß das Gesamtpräparat langzeitbeständig ist. Beim Auftragen auf die Haut wird zwar die Emulsion gebrochen, jedoch werden die beiden Wirkstoffe von der Haut in unterschiedlicher Weise so schnell resorbiert, daß es trotz der gemeinsamen Auftragung nicht zur Denaturierung des Collagenpräparates kommt.

In entsprechender Weise können auch an unterschiedlichen Orten wirksame Arzneimittel in verschiedenen X/O-Phasen verteilt werden. Auf diese Weise wird es beispielsweise möglich, in einen gemeinsamen flüssigen Träger pharmazeutische Wirkstoffe einzubringen, die an unterschiedlichen Stellen des Verdauungstraktes zur Wirkung kommen sollen. Dies wird dadurch erreicht, daß zur Herstellung der verschiedenen X/O-Phasen Emulgatoren eingesetzt werden, die unter den Bedingungen des jeweiligen Wirkungsortes nicht beständig sind. Dadurch werden dann die in den Tropfen der X/O-Phasen enthaltenen Wirkstoffe freigesetzt.

Die Ölphase und/oder die X-Komponente können Feststoffe wie Pigmente, Mikrosphären, Silicagel oder Wachs enthalten. Pigmente können als Lichtschutzfilter (zum Beispiel in der Kosmetik) dienen, Mikrosphären oder Silicagel können als Träger von Wirkstoffen eingesetzt werden, und Wachs wird als Grundstoff von zum Beispiel Polituren verwendet.

Selbstverständlich ist es bei der Herstellung von Emulsionen mit Wirkstoffen der vorgenannten Art ohne weiteres möglich, diesen Emulsionen außer den Wirkstoffen selbst auch die sonstigen, nach dem Anwendungszweck jeweils erforderlichen Hilfs- und Zusatzstoffe hinzuzufügen.

## Patentansprüche

1. Stabile multiple Emulsion vom X/O/Y-Typ, bei der X eine mit Öl nicht mischbare Komponente, O eine Ölphase und Y eine wässrige Phase sind und deren Emulgator oder Emulgatoren ausschließlich ausgewählt sind aus der Gruppe W/O-Emulgatoren, Emulgatoren mit einem HLB-Wert ≤ 6 und deren Mischung, **dadurch gekennzeichnet, daß** die wässrige Phase ein wässriges Gel bildet, wobei das Gel von einem ein flüssigkristallines Netzwerk bildenden Emulgator gebildet ist, und daß die X-Komponente ein mit Wasser mischbares Polyol ist.

2. Stabile multiple Emulsion nach Anspruch 1, **dadurch gekennzeichnet, daß** der Emulgator aus der Gruppe Glycerinester, Sorbitanester, Sorbitolester, Polyglycerinester, Fettalkohole, Propylenglycolester, Alkylglucosidester, Zuckerester, Lecithin, Siliconcopolymere und deren Mischungen oder Derivaten ausgewählt ist.

3. Stabile multiple Emulsion nach Anspruch 2, **dadurch gekennzeichnet, daß** die Ester von langkettigen gesättigten Fettsäuren oder einem bei Raumtemperatur festen Gemisch von langkettigen gesättigten und ungesättigten Fettsäuren gebildet sind.

4. Stabile multiple Emulsion nach einem der Ansprüche 1 bis 3, **dadurch gekennzeichnet, daß** die Ölphase aus der Gruppe Siliconöle, Paraffinöle, Triglyceride, Fettalkohole, Esteröle oder deren Mischung ausgewählt ist.

5. Stabile multiple Emulsion nach Anspruch 4, **dadurch gekennzeichnet, daß** die Ölphase einen Feststoff enthält, der aus der Gruppe Pigmente, Mikrosphären, Silicagel, Wachs ausgewählt ist.

6. Stabile multiple Emulsion nach einem der Ansprüche 1 bis 5, **dadurch gekennzeichnet, daß** das Polyol aus der Gruppe Propylenglycol, Butylenglycol, Polyalkylenglycol, Glycerin, Polyglycerin oder deren Mischung ausgewählt ist.

7. Stabile multiple Emulsion nach einem der Ansprüche 1 bis 6, **dadurch gekennzeichnet, daß** die X-Komponente einen Wirkstoff enthält, der aus der Gruppe pharmazeutischer, kosmetischer, waschmitteltechnischer, lebensmitteltechnologischer oder agrartechnologischer Wirkstoffe ausgewählt ist.

8. Stabile multiple Emulsion nach einem der Ansprüche 1 bis 7, **dadurch gekennzeichnet, daß** die X-Komponente einen Feststoff enthält, der aus der Gruppe Pigmente, Mikrosphären, Silicagel, Wachs ausgewählt ist.

9. Stabile multiple Emulsion nach einem der Ansprüche 1 bis 8, **dadurch gekennzeichnet, daß** mehrere X-Komponenten vorgesehen und jeweils in einer Ölphase dispergiert sind und daß die so gebildeten, unterschiedlichen X/O-Phasen bleibend voneinander getrennt nebeneinander in der X/O/Y-Emulsion enthalten sind.

10. Stabile multiple Emulsion nach einem der vorstehenden Ansprüche, **dadurch gekennzeichnet, daß** die X/O-Phase aus Tropfen mit einem Durchmesser im Bereich von 1 bis 10 µm gebildet ist.

11. Verfahren zur vereinfachten Herstellung einer stabilen multiplen Emulsion vom X/O/Y-Typ nach einem der Ansprüche 1 bis 10, **dadurch gekennzeichnet, daß** eine in an sich bekannter Weise hergestellte X/O-Phase und die wässrige Phase getrennt auf eine Temperatur im Bereich von 20°C bis 90°C erwärmt werden, daß die erwärmte X/O-Phase und die erwärmte wässrige Phase unter Rühren zusammengegeben werden und die so erhaltene Mischung auf Raumtemperatur abgekühlt wird.

12. Verfahren nach Anspruch 11, **dadurch gekennzeichnet, daß** die X/O-Phase und die wässrige Phase getrennt auf eine Temperatur im Bereich von 60°C bis 80°C erwärmt werden.

13. Verfahren nach Anspruch 11 oder 12, **dadurch gekennzeichnet, daß** eine erste X/O/Y-Emulsion auf eine Temperatur im Bereich von 20°C bis 90°C erwärmt und unter Rühren mit einer zweiten, in gleicher Weise hergestellten X/O/Y-Emulsion vermischt und die so erhaltene Mischung auf Raumtemperatur abgekühlt wird.

14. Verfahren nach Anspruch 13, **dadurch gekennzeichnet, daß** die erste X/O/Y-Emulsion auf eine Temperatur im Bereich von 50°C bis 60°C erwärmt wird.

15. Verwendung von mindestens einem Emulgator, der aus der Gruppe W/O-Emulgatoren, Emulgatoren mit einem HLB-Wert von ≤ 6 und deren Mischung ausgewählt ist, zur Herstellung einer langzeitstabilen multiplen X/0/Y-Emulsion nach einem der Ansprüche 1 bis 10.

## Claims

1. Stable multiple emulsion of the X/O/Y type, in which X is a component which is immiscible with oil, O is an oil phase and Y is an aqueous phase, and the emulsifier or emulsifiers thereof are exclusively selected from the group consisting of W/O emulsifiers, emulsifiers having an HLB value s 6 and mixtures thereof, **characterised in that** the aqueous phase forms an aqueous gel, wherein the gel is formed by an emulsifier which forms a liquid-crystalline network, and **in that** the X component is a polyol which is miscible with water.

2. Stable multiple emulsion according to claim 1, **characterised in that** the emulsifier is selected from the group consisting of glycerol esters, sorbitan esters, sorbitol esters, polyglycerol esters, fatty alcohols, propylene glycol esters, alkyl glucoside esters, sugar esters, lecithin, silicone copolymers and mixtures or derivatives thereof.

3. Stable multiple emulsion according to claim 2, **characterised in that** the esters are formed of long-chain saturated fatty acids or a mixture of long-chain saturated and unsaturated fatty acids which is solid at room temperature.

4. Stable multiple emulsion according to one of claims 1 to 3, **characterised in that** the oil phase is selected from the group consisting of silicone oils, paraffin oils, triglycerides, fatty alcohols, ester oils or mixtures thereof.

5. Stable multiple emulsion according to claim 4, **characterised in that** the oil phase contains a solid selected from the group consisting of pigments, microspheres, silica gel, wax.

6. Stable multiple emulsion according to one of claims 1 to 5, **characterised in that** the polyol is selected from the group consisting of propylene glycol, butylene glycol, polyalkylene glycol, glycerol, polyglycerol or mixtures thereof.

7. Stable multiple emulsion according to one of claims 1 to 6, **characterised in that** the X component contains an active agent selected from the group consisting of pharmaceutical active agents, cosmetic active agents, and active agents relating to washing, food or agricultural technology.

8. Stable multiple emulsion according to one of claims 1 to 7, **characterised in that** the X component contains a solid selected from the group consisting of pigments, microspheres, silica gel, wax.

9. Stable multiple emulsion according to one of claims 1 to 8, **characterised in that** a plurality of X components are provided and are in each case dispersed in an oil phase, and **in that** the different X/O phases thus formed are contained next to one another in the X/O/Y emulsion while remaining separate from one another.

10. Stable multiple emulsion according to one of the preceding claims, **characterised in that** the X/O phase is formed of drops having a diameter in the range from 1 to 10 µm.

11. Method for the simplified preparation of a stable multiple emulsion of the X/O/Y type according to one of claims 1 to 10, **characterised in that** an X/O phase prepared in a known manner and the aqueous phase are separately heated to a temperature in the range from 20°C to 90°C, **in that** the heated X/O phase and the heated aqueous phase are combined with stirring and the mixture thus obtained is cooled to room temperature.

12. Method according to claim 11, **characterised in that** the X/O phase and the aqueous phase are separately heated to a temperature in the range from 60°C to 80°C.

13. Method according to claim 11 or 12, **characterised in that** a first X/O/Y emulsion is heated to a temperature in the range from 20°C to 90°C and mixed with stirring with a second X/O/Y emulsion which has been prepared in the same way, and the mixture thus obtained is cooled to room temperature.

14. Method according to claim 13, **characterised in that** the first X/O/Y emulsion is heated to a temperature in the range from 50°C to 60°C.

15. Use of at least one emulsifier selected from the group consisting of W/O emulsifiers, emulsifiers having an HLB value s 6 and mixtures thereof, for preparing a long-term stable multiple X/O/Y emulsion according to one of claims 1 to 10.

## Revendications

1. Emulsion multiple stable de type X/O/Y pour laquelle X est un composant non miscible avec de l'huile, O une phase d'huile et Y une phase aqueuse et dont l'émulsifiant ou les émulsifiants sont choisis exclusivement parmi le groupe des émulsifiants d'eau en huile, des émulsifiants ayant une valeur d'équilibre hydrophile/lipophile (HLB) ≤ 6 et de leur mélange, **caractérisée en ce que** la phase aqueuse forme un gel aqueux, le gel étant formé par un émulsifiant formant un réseau de cristaux liquides et **en ce que** le composant X est un polyol miscible à l'eau.

2. Emulsion multiple stable selon la revendication 1, **caractérisée par le fait que** l'émulsifiant est choisi parmi le groupe des esters glycériques, des esters de sorbitane, des esters de sorbitol, des esters polyglycériques, des alcools gras, des esters de propylèneglycol, des esters d'alkylglucoside, des esters de sucre, de la lécithine, des copolymères de silicone et de leurs mélanges ou de leurs dérivés.

3. Emulsion multiple stable selon la revendication 2, **caractérisée par le fait que** les esters sont formés d'acides gras saturés à longues chaînes ou d'un mélange consistant à température ambiante d'acides gras non saturés ou saturés à longues chaînes.

4. Emulsion multiple stable selon l'une des revendications 1 à 3, **caractérisée par le fait que** la phase d'huile est choisie parmi le groupe des huiles de silicone, des huiles de paraffine, des triglycérides, des alcools gras, des huiles d'esters ou de leur mélange.

5. Emulsion multiple stable selon la revendication 4, **caractérisée par le fait que** la phase d'huile contient une matière solide choisie parmi le groupe des pigments, des microsphères, du silicagel, de la cire.

6. Emulsion multiple stable selon les revendications 1 à 5, **caractérisée par le fait que** le polyol est choisi parmi le groupe de propylèneglycol, du butylèneglycol, des polyalkylèneglycols, de la glycérine, des polyglycérines ou de leur mélange.

7. Emulsion multiple stable selon les revendications 1 à 6, **caractérisée par le fait que** le composant X comprend une matière active choisie parmi le groupe des matières actives pharmaceutiques, cosmétiques, détergentes, de l'industrie alimentaire ou de l'industrie agricole.

8. Emulsion multiple stable selon l'une des revendications 1 à 7, **caractérisée par le fait que** le composant X comprend une matière solide choisie parmi le groupe des pigments, des microsphères, du gel de silice et de la cire.

9. Emulsion multiple stable selon l'une des revendications 1 à 8, **caractérisée par le fait que** plusieurs composants X sont prévus et qu'ils sont dispersés chaque fois dans une phase d'huile et que les diverses phases X/O ainsi formées sont contenues les unes à côté des autres dans l'émulsion X/O/Y en restant séparées les unes des autres.

10. Emulsion multiple stable selon l'une des revendications précédentes, **caractérisée par le fait que** la phase X/O est formée de gouttes ayant un diamètre situé dans le domaine allant de 1 à 10 µm.

11. Procédé destiné à fabriquer de façon simplifiée une émulsion stable multiple de type X/O/Y selon l'une des revendications 1 à 10, **caractérisé par le fait qu'**une phase X/O fabriquée de façon connue en soi et la phase aqueuse sont réchauffées séparément jusqu'à ce qu'elles atteignent une température située dans le domaine allant de 20°C à 90°C, que la phase X/O réchauffée et la phase aqueuse réchauffée sont unies en les remuant et que le mélange ainsi obtenu est refroidi jusqu'à atteindre la température ambiante.

12. Procédé selon la revendication 11, **caractérisé par le fait que** la phase X/O et la phase aqueuse sont réchauffées séparément jusqu'à ce qu'elles atteignent une température située dans le domaine de 60°C-80°C.

13. Procédé selon la revendication 11 ou 12, **caractérisé par le fait qu'**une première émulsion X/O/Y est réchauffée jusqu'à ce qu'elle atteigne une température située dans le domaine allant de 20°C à 90°C et qu'elle est mélangée en la remuant à une seconde émulsion X/O/Y fabriquée de la même façon et le mélange ainsi obtenu est refroidi jusqu'à atteindre la température ambiante.

14. Procédé selon la revendication 13, **caractérisé par le fait que** la première émulsion X/O/Y est réchauffée jusqu'à ce qu'elle atteigne une température située dans le domaine de 50°C-60°C.

15. Utilisation d'au moins un émulsifiant choisi parmi le groupe des émulsifiants d'eau dans l'huile, des émulsifiants ayant une valeur d'équilibre hydrophile/lipophile (HLB) ≤ 6 et de leur mélange, destinée à fabriquer une émulsion X/O/Y multiple et stable à long terme selon l'une des revendications 1 à 10.
